# EUROPEAN PATENT APPLICATION

(11) **EP 4 799 657 A1**
(43) Date of publication of application: **02.09.2026**
(21) Application number: 25200898.2
(22) Date of filing: 08.09.2025
(51) Int. Cl.: A61L 27/24, A61L 27/50, A61L 27/52, A61L 27/58, A61L 31/04, A61L 31/14, A61L 31/16

(54) **IMPLANTABLE RECOMBINANT COLLAGEN MICROPARTICLE AND PREPARATION METHOD THEREFOR**

(30) Priority: 28.02.2025 CN 202510238767
(71) Applicant: WitKang Zhiyuan Medical Devices (Xi'an) Co., Ltd., Xi'an City Shaanxi 710075 (CN)
(72) Inventor: WANG, Meihua, Xi'an City, 710075 (CN); LI, Yanting, Xi'an City, 710075 (CN); CHEN, Liang, Xi'an City, 710075 (CN); HOU, Jianing, Xi'an City, 710075 (CN); WANG, Jiuna, Xi'an City, 710075 (CN); WANG, Ya, Xi'an City, 710075 (CN); LI, Yuan, Xi'an City, 710075 (CN)
(74) Representative: IP TRUST SERVICES

(57) **Abstract**

The present disclosure discloses an implantable recombinant collagen microparticle and a preparation method therefor. At present, although materials used in the field of soft tissue filling, such as animal collagen and sodium hyaluronate, have good effects, the materials still have problems such as low biosafety, residual cross-linking agents, and a short maintenance time. In order to solve the above problems of existing medical aesthetic materials, the present disclosure prepares an implantable recombinant collagen microparticle by using a method of self-assembly combined with physical cross-linking. The microparticle becomes an injectable milky white opaque or colorless and transparent gel after being completely swollen with a water solvent. Compared with traditional medical aesthetic fillers, the gel prepared by the present disclosure has the characteristics of a good biocompatibility, a long degradation period, a simple preparation process, easy mass production, etc.

## Description

### TECHNICAL FIELD

The present disclosure belongs to the field of biomedical materials, and particularly relates to an implantable recombinant collagen microparticle and a preparation method therefor.

### BACKGROUND

A hydrogel is a polymer material having a three-dimensional network structure with water as a dispersion medium. It has good biocompatibility, a porous structure, permeability, and hydrophilicity, can simulate a natural extracellular matrix, providing a suitable microenvironment for cells, and has been increasingly extensively used in tissue repair and regeneration. Hydrogels used in the biomedical field can be categorized into natural hydrogels and artificially synthesized hydrogels according to their sources. Natural hydrogels include collagen, gelatin, hyaluronic acid, chitosan, etc., which have good biocompatibility and biodegradability but poor mechanical properties, and are limited by the potential immunogenicity in foreign hosts. Artificially synthesized hydrogels, such as polyethylene glycol derivatives, polycaprolactone, and polyvinyl alcohol, have the characteristics of a stable composition, a controllable structure, a low immunogenicity, etc., and can withstand a relatively high mechanical load, thereby becoming a research hotspot. A hydrogel can be used as a carrier scaffold to provide a regeneration template or matrix. On the one hand, cells can adhere thereto and proliferate, have their responses coordinated, and thus allow damaged tissue to be regenerated after injury. On the other hand, extra stimulation by drugs, cytokines, stem cells, etc., loaded thereon can promote tissue regeneration. At present, natural polymer materials used for preparing hydrogels are mainly animal collagen, sodium hyaluronate, etc.

Collagen is a biopolymer, which is the most abundant and widely distributed functional protein in mammals and accounts for 25%-30% of the total amount of proteins. Collagen is widely used in fields such as medicine, tissue engineering, and cosmetics due of its good biocompatibility, biodegradability, and biological activity. However, the commonly used collagen is derived from animal tissues such as pigskin, cowhide, and bovine tendon, and animal collagen may have residual viruses and cause immune rejection. Therefore, for some users, there are still rejection problems such as redness and swelling, allergy and chronic inflammation at the use site.

Hydrogels prepared by a chemical cross-linking method have a better stability and a higher flexibility. However, chemical cross-linking agents, such as 1,4-butanediol diglycidyl ether and sodium β-glycerophosphate, are needed during cross-linking, and incomplete residue removal will cause harm to the human body. Enzymes, as biomacromolecules, widely exist in organisms. They are safe and non-toxic, have high catalytic efficiency, and can facilitate the formation of stable chemical bonds. Compared with traditional cross-linking agents, enzymes are more suitable for the human body. However, enzymes are often expensive, which may be a limiting factor for large-scale application, and the stability of enzymes is relatively poor, which may affect the efficiency of cross-linking reaction and the consistency of products.

In order to avoid problems of cytotoxicity related to chemical cross-linking agents and cost, a physical cross-linking method can be used, which can prevent exogenous substances from entering collagen but result in a low degree of cross-linking.

### SUMMARY

An objective of the present disclosure is to prepare an implantable recombinant collagen microparticle to overcome the shortcomings of existing hydrogels and soft tissue filling materials. The implantable recombinant collagen microparticle is obtained by a method of self-assembly combined with physical cross-linking with one raw material, i.e. recombinant collagen. By this method, the pH of the recombinant collagen solution is changed, so that the recombinant collagen microparticle having a rod-like structure or spherical structure can be obtained. The obtained material has safety, non-toxicity, biodegradability, excellent biocompatibility, and no viral hazard. The method of the present disclosure not only avoids introducing new chemical substances, thereby avoiding the residue of any chemical reagent, thus providing a better biosafety, but also involves self-assembly using the unique self-assembly ability of the recombinant collagen raw material, in combination with physical cross-linking, to achieve a higher degree of cross-linking. Specifically, the present disclosure adopts the following technical solutions:

In a first aspect, the present disclosure provides a method for preparing an implantable recombinant collagen microparticle, comprising:
(1) dissolving a recombinant collagen in water to obtain a recombinant collagen solution;
(2) drying and granulating the recombinant collagen solution to obtain a first recombinant collagen microparticle; and
(3) subjecting the first recombinant collagen microparticle to dry and thermal cross-linking to obtain a second recombinant collagen microparticle.

In some embodiments, in step (1), a certain amount of the recombinant collagen is dissolved in water and stirred until it is completely dissolved and the solution is transparent, thus obtaining a recombinant collagen aqueous solution. The recombinant collagen can be quickly dispersed into a nano-scale particle size in water and has a super strong hydrophilicity. The exterior of the collagen molecule has regular hydrophilic groups, leading to a super strong aggregation ability, and the interior of the collagen molecule has hydrophobic groups, which can form a micro-scaffold.

In some embodiments, in step (2), the recombinant collagen solution is dried and granulated by using a spray drier.

In some embodiments, step (2) further comprises adjusting the pH of the recombinant collagen solution to less than 7 or more than 7.

In some embodiments, the first recombinant collagen microparticle has a rod-like structure or a spherical structure.

In some embodiments, the pH of the recombinant collagen solution is adjusted to less than 7, and the first recombinant collagen microparticle has a spherical structure.

In some embodiments, the diameter of the spherical structure is 2-10 µm, and the interior of the spherical structure is a porous network structure.

In some embodiments, the pH of the recombinant collagen solution is adjusted to more than 7, and the first recombinant collagen microparticle has a rod-like structure.

In some embodiments, the diameter of the rod-like structure is 2-5 µm.

In the present disclosure, a spray drier is used for drying, and during the drying process, due to the special structure of the recombinant collagen raw material used, the raw material can spontaneously assemble into a micro-scaffold and self-aggregate to form a microparticle structure; therefore, during the heating and drying process, protein chains quickly lose water therebetween, approach each other, and form a large number of hydrogen bonds, thus enhancing the self-assembly effect of the recombinant collagen. The self-assembly of the recombinant collagen is completed during this process, and the recombinant collagen microparticle is in a sol state after being dissolved in water.

In some embodiments, the drying temperature in the spray drier is 100-200°C, for example, it can be 120°C, 130°C, 140°C, 150°C, 170°C, 180°C, etc., and further preferably, the drying temperature in the spray drier is 120-190°C.

In some embodiments, in step (3), the dry and thermal cross-linking is carried out in a vacuum drying oven.

In the present disclosure, the first recombinant collagen microparticle is subjected to dry and thermal cross-linking in the vacuum drying oven. By increasing the denaturation temperature of the collagen, i.e., the temperature at which the helical structure unwinds, and dehydrating the collagen, cross-linking occurs between the collagen molecules, so that the mechanical properties of the collagen can be significantly improved to obtain a second recombinant collagen microparticle with a high strength and resistance to degradation.

In some embodiments, the temperature for the dry and thermal cross-linking in the vacuum drying oven is 90-250°C, for example, it can be 100°C, 140°C, 180°C, 200°C, 240°C, etc., and further preferably, the temperature of the vacuum drying oven is 100-240°C.

In some embodiments, the time for the dry and thermal cross-linking in the vacuum drying oven is 1-10 h, for example, it can be 2 h, 3 h, 4 h, 5 h, 6 h, 8 h, 9 h, etc., and further preferably, the time in the vacuum drying oven is 2-9 h.

The recombinant collagen of the present disclosure is a recombinant human-derived collagen disclosed in CN 108070032B [METHOD FOR PURIFYING RECOMBINANT HUMAN-DERIVED COLLAGEN].

In some embodiments, the amino acid sequence of the recombinant collagen is as set forth in SEQ ID No: 1.

The recombinant collagen raw materials described in the specific embodiments of the present disclosure are all proteins with the amino acid residue sequence as set forth in SEQ ID No: 1 in the sequence listing.

In some embodiments, the recombinant human-derived collagen is obtained by preparing a plasmid, mixing the plasmid with *Pichia pastoris* for transformation, screening for multi-copy insertion recombinants, fermentation, and purification. This collagen has a good biocompatibility and a purity of 95% or more. Since the structure and function thereof are very similar to the autologous collagen in the human body, the use of this collagen can effectively solve the problem of viral hazards in extracting collagen from animal tissues. In addition, the recombinant collagen can be quickly dispersed into a nano-scale particle size in water and has a super strong hydrophilicity. The exterior of the collagen molecule has regular hydrophilic groups, leading to a super strong aggregation ability, and the interior of the collagen molecule has hydrophobic groups, which can form a micro-scaffold. Therefore, the recombinant collagen has excellent self-assembly ability.

In some embodiments, the concentration of the recombinant collagen solution is 5-20% (wt), for example, it can be 5% (wt), 7% (wt), 10% (wt), 12% (wt), 15% (wt), 18% (wt), 20% (wt), etc., and further preferably, the concentration of the recombinant collagen solution is 8-15% (wt).

In some embodiments, the method further comprises adding an aqueous phase to the second recombinant collagen microparticle (implantable recombinant collagen microparticle) to allow swelling, to obtain recombinant collagen microparticles in gel form(recombinant collagen gels).

In some embodiments, the gel form is a milky white opaque gel or a colorless and transparent jelly-like gel.

In some embodiments, the pH of the recombinant collagen solution is adjusted to less than 7, and the first recombinant collagen microparticle has a spherical structure. After the aqueous phase is added to the second recombinant collagen microparticle (implantable recombinant collagen microparticle) for swelling, recombinant collagen microparticles existing as milky white opaque gels are obtained.

In some embodiments, the pH of the recombinant collagen solution is adjusted to more than 7, and the first recombinant collagen microparticle has a rod-like structure. After the aqueous phase is added to the second recombinant collagen microparticle (implantable recombinant collagen microparticle) for swelling, recombinant collagen microparticles existing as colorless and transparent jelly-like gels are obtained.

In some embodiments, after the aqueous phase is added to the second recombinant collagen microparticle, complete swelling is required.

In some embodiments, the swelling time required for the complete swelling is 10-15 h.

In some embodiments, the aqueous phase is selected from one or more of ultrapure water, water for injection, and a phosphate buffer, preferably water for injection.

The recombinant collagen microparticle obtained by the two processes, i.e. self-assembly and dry and thermal cross-linking of the recombinant collagen, is a water-insoluble implantable recombinant collagen microparticle. After a certain amount of a water solvent is added, the recombinant collagen microparticles are completely swollen into recombinant collagen microparticles existing as milky white opaque gels or recombinant collagen microparticles existing as colorless and transparent jelly-like gels, both of which are referred to as recombinant collagen microparticles in gel form. In some embodiments, the concentration of the recombinant collagen microparticles in gel form is 5-15% (wt), for example, it can be 5% (wt), 8% (wt), 10% (wt), 12% (wt), 15% (wt), etc., and further preferably, the concentration of the recombinant collagen microparticles in gel form is 8-13% (wt).

In some embodiments, the method further comprises:adding an aqueous phase to the second recombinant collagen microparticle (implantable recombinant collagen microparticle), stirring the mixture at a rotating speed of 100-150 r/min for 1-4 h, and then allowing the mixture for swelling at a temperature of 2-8°C for 10-15 h to obtain the recombinant collagen microparticles in gel form.

In some embodiments, the method further comprises sterilizing the recombinant collagen microparticles in gel form.

In some embodiments, the sterilization conditions are: moist heat sterilization at 120°C for 30 min in a high-temperature and high-pressure steam sterilizer.

In a second aspect, the present disclosure provides an implantable recombinant collagen microparticle, wherein the amino acid sequence of a recombinant collagen in the recombinant collagen microparticle is as set forth in SEQ ID No: 1.

In some embodiments, the implantable recombinant collagen microparticle is prepared by the preparation method according to the first aspect of the present disclosure.

In some embodiments, the recombinant collagen microparticle exists in gel form upon addition of an aqueous phase.

In some embodiments, the pH value of the recombinant collagen microparticles in gel form is 5-7.

In the present disclosure, the milky white opaque gel obtained after the implantable recombinant collagen microparticle (spherical structure) obtained by the method for preparing the implantable recombinant collagen microparticle according to the present disclosure are completely swollen, can maintain in vivo for 6 months or more after injection and have a relatively long degradation period. In addition, after being injected into a tissue, it is less prone to displacement and has a relatively strong supporting performance. The colorless and transparent jelly-like gel which is obtained after the implantable recombinant collagen microparticle (rod-like structure) obtained by the method of the present application are completely swollen, may also be used for medical aesthetic plastic surgery such as water light injection therapy.

In a third aspect, the present disclosure provides a method for medical aesthetic filling, medical aesthetic plastic surgery, soft tissue repair, or adhesion prevention, the method comprising: injecting the recombinant collagen microparticle according to the second aspect of the present disclosure into the skin, or contacting the recombinant collagen microparticle with the skin.

In a fourth aspect, the present disclosure provides an implantable recombinant collagen microparticle according to the second aspect of the present disclosure for use in the preparation of a medical material.

In some embodiments, the medical material is used for medical aesthetic filling, soft tissue repair, or adhesion prevention.

### Beneficial Effects:

Compared with the traditional chemical cross-linking preparation method, the material prepared using the implantable recombinant collagen microparticle prepared by the method of the present application has no toxicity and no residue and contains the only one recombinant collagen raw material, leading to a high safety. Compared with the traditional preparation method of enzymatic cross-linking (TG enzyme, etc.), it has a higher cross-linking rate and a lower cost. The method of self-assembly combined with physical cross-linking can achieve a higher degree of cross-linking and also overcome the shortcomings of a low degree of cross-linking in physical cross-linking. After the implantable recombinant collagen microparticle (spherical structure) prepared by the method of the present application become a milky white opaque gel after being completely swollen. The gel enters the target position by injection, plays a relatively strong supporting role, and is less prone to displacement. It can maintain for 6 months or more in vivo, with a relatively long degradation period and good biocompatibility. The implantable recombinant collagen microparticle (rod-like structure) prepared by the method of the present application can become a colorless and transparent jelly-like gel after being completely swollen, which also has a relatively good application potential in medical aesthetic plastic surgery such as water light injection therapy. The preparation process is simple and easy for mass production, and it is very suitable for fields such as medical aesthetic filling, medical aesthetic plastic surgery, soft tissue repair, and adhesion prevention.

### BRIEF DESCRIPTION OF THE DRAWINGS

FIG. 1 shows appearance images of recombinant collagen gels;
FIG. 2 shows a transmission electron microscope image of a recombinant collagen raw material (panel a of FIG. 2), a scanning electron microscope image of an implantable recombinant collagen microparticle (spherical structure) (panel b of FIG. 2), and a scanning electron microscope image of an implantable recombinant collagen microparticle (rod-like structure) (panel c of FIG. 2);
FIG. 3 shows hematoxylin-eosin staining images of subcutaneous tissues of the back of rats;
FIG. 4 shows a schematic diagram of the swelling performance of an implantable recombinant collagen microparticle;
FIG. 5 shows a schematic diagram of the pushing force for an implantable recombinant collagen microparticle; and
FIG. 6 shows schematic diagrams of the degradation of an implantable recombinant collagen microparticle in vivo.

### DESCRIPTION OF THE EMBODIMENTS

Before further describing the specific embodiments of the present disclosure, it should be understood that the scope of protection of the present disclosure is not limited to the following specific embodiments. It should also be understood that the terminology used in the embodiments of the present disclosure is for the purpose of describing specific embodiments, rather than limiting the scope of protection of the present disclosure.

When numerical ranges are given in embodiments, it should be understood that unless otherwise specified in the present disclosure, two endpoints of each numerical range and any numerical value between the two endpoints can both be selected. Unless otherwise defined, all technical and scientific terms used in the present disclosure have the same meanings as commonly understood by those skilled in the art. In addition to the specific methods, equipment, and materials used in the embodiments, any methods, equipment, and materials in the prior art similar to or equivalent to the methods, equipment, and materials in the embodiments of the present disclosure can also be used according to the mastery of the prior art by those skilled in the art and the disclosure of the present disclosure to realize the present disclosure.

Unless otherwise specified, the experimental methods, detection methods and preparation methods not described in detail in the present disclosure all use conventional technologies in the art.

The recombinant collagen used in the following examples is available from Shaanxi WitKang Bio-tech Co., Ltd., and the amino acid sequence of the recombinant collagen is: GPPGEPGNPGKPGSPGPAGSNGEPGPAGSPGEKGSQGSNGNPGPAGNQGQPGNKGSPGN PGKPGEPGSNGPQGEPGSQGNPGKNGQPGSPGSQGSPGNQGQPGKPGQPGEQGSPGNQ GPAGNEGPKGQPGQNGKPGSPGPPGEPGNPGKPGSPGPAGSNGEPGPAGSPGEKGSQGS NGNPGPAGNQGQPGNKGSPGNPGKPGEPGSNGPQGEPGSQGNPGKNGQPGSPGSQGSP GNQGQPGKPGQPGEQGSPGNQGPAGNEGPKGQPGQNGKPGTPGPPGEPGNPGKPGSPG PAGSNGEPGPAGSPGEKGSQGSNGNPGPAGNQGQPGNKGSPGNPGKPGEPGSNGPQGE PGSQGNPGKNGQPGSPGSQGSPGNQGQPGKPGQPGEQGSPGNQGPAGNEGPKGQPGQN GKP (SEQ ID No: 1). Other used materials, preparations, etc. can all be obtained from commercial channels unless otherwise specified.

### Example 1

Step 1: 50 g of a recombinant collagen raw material (a protein with an amino acid sequence as set forth in SEQ ID No: 1) was weighed, dissolved in 450 g of water for injection, and stirred for 4 hours at room temperature until it was completely dissolved and provided a colorless and transparent solution, and the pH was adjusted to be acidic (pH = 5.24) to obtain a 10% (wt) recombinant collagen solution;
Step 2: the above solution was dried and granulated by a spray drier at 120°C to obtain a first recombinant collagen microparticle having a spherical structure with a diameter of 2-10 µm;
Step 3: the first recombinant collagen microparticle having a spherical structure was subjected to dry and thermal cross-linking for 2 h in a vacuum drying oven at 100°C to obtain a second recombinant collagen microparticle having a spherical structure with high strength (implantable recombinant collagen microparticle); and
Step 4: 1 g of the second recombinant collagen microparticle having a spherical structure (implantable recombinant collagen microparticle), which was obtained by the two processes, i.e. self-assembly and dry and thermal cross-linking, was weighed, 12.5 g of water for injection was added, the microparticles were swollen for 12 h in a refrigerator at 4°C, and the mixture was stirred for 2 h at a rotating speed of 120 r/min to obtain a milky white opaque recombinant collagen gel at a concentration of 8% (wt) and a pH of 6.7.

### Example 2

Step 1: 60 g of a recombinant collagen raw material (a protein with an amino acid sequence as set forth in SEQ ID No: 1) was weighed, dissolved in 440 g of water for injection, and stirred for 4 hours at room temperature until it was completely dissolved and provided a colorless and transparent solution, and the pH was adjusted to be acidic (pH = 4.85) to obtain a 12% (wt) recombinant collagen solution;
Step 2: the above solution was dried and granulated by a spray drier at 140°C to obtain a first recombinant collagen microparticle having a spherical structure with a diameter of 2-10 µm;
Step 3: the first recombinant collagen microparticle having a spherical structure was subjected to dry and thermal cross-linking for 3 h in a vacuum drying oven at 120°C to obtain a second recombinant collagen microparticle having a spherical structure with a high strength (implantable recombinant collagen microparticle); and
Step 4: 1 g of the second recombinant collagen microparticle having a spherical structure (implantable recombinant collagen microparticle), which was obtained by the two processes, i.e. self-assembly and dry and thermal cross-linking, was weighed, 10 g of water for injection was added, and the microparticles were swollen for 12 h in a refrigerator at 4°C, and the mixture was stirred for 2 h at a rotating speed of 120 r/min to obtain a milky white opaque recombinant collagen gel with a concentration of 10% (wt) and a pH of 6.9.

### Example 3

Step 1: 65 g of a recombinant collagen raw material (a protein with an amino acid sequence as set forth in SEQ ID No: 1) was weighed, dissolved in 435 g of water for injection, and stirred for 4 hours at room temperature until it was completely dissolved and provided a colorless and transparent solution, and the pH was adjusted to be acidic (pH = 5.32) to obtain a 13% (wt) recombinant collagen solution;
Step 2: the above solution was dried and granulated by a spray drier at 165°C to obtain a first recombinant collagen microparticle having a spherical structure with a diameter of 2-10 µm;
Step 3: the first recombinant collagen microparticle having a spherical structure was subjected to dry and thermal cross-linking for 4 h in a vacuum drying oven at 135°C to obtain a second recombinant collagen microparticle having a spherical structure with a high strength (implantable recombinant collagen microparticle); and
Step 4: 1.1 g of the second recombinant collagen microparticle having a spherical structure (implantable recombinant collagen microparticle), which was obtained by the two processes, i.e. self-assembly and dry and thermal cross-linking, was weighed, 9.9 g of water for injection was added, the microparticles were swollen for 12 h in a refrigerator at 4°C, and the mixture was stirred for 2 h at a rotating speed of 120 r/min to obtain a milky white opaque recombinant collagen gel with a concentration of 11% (wt) and a pH of 6.6.

### Example 4

Step 1: 70 g of a recombinant collagen raw material (a protein with an amino acid sequence as set forth in SEQ ID No: 1) was weighed, dissolved in 430 g of water for injection, and stirred for 4 hours at room temperature until it was completely dissolved and provided a colorless and transparent solution, and the pH was adjusted to be acidic (pH = 4.62) to obtain a 14% (wt) recombinant collagen solution;
Step 2: the above solution was dried and granulated by a spray drier at 175°C to obtain a first recombinant collagen microparticle having a spherical structure with a diameter of 2-10 µm;
Step 3: the first recombinant collagen microparticle having a spherical structure was subjected to dry and thermal cross-linking for 3 h in a vacuum drying oven at 170°C to obtain a second recombinant collagen microparticle having a spherical structure with a high strength (implantable recombinant collagen microparticle); and
Step 4: 1.2 g of the second recombinant collagen microparticle having a spherical structure (implantable recombinant collagen microparticle), which was obtained by the two processes, i.e. self-assembly and dry and thermal cross-linking, was weighed, 9.8 g of water for injection was added, the microparticles were swollen for 12 h in a refrigerator at 4°C, and the mixture was stirred for 2 h at a rotating speed of 120 r/min to obtain a milky white opaque recombinant collagen gel with a concentration of 12% (wt) and a pH of 6.8.

### Example 5

Step 1: 75 g of a recombinant collagen raw material (a protein with an amino acid sequence as set forth in SEQ ID No: 1) was weighed, dissolved in 425 g of water for injection, and stirred for 4 hours at room temperature until it was completely dissolved and provided a colorless and transparent solution, and the pH was adjusted to be acidic (pH = 4.287) to obtain a 15% (wt) recombinant collagen solution;
Step 2: the above solution was dried and granulated by a spray drier at 180°C to obtain a first recombinant collagen microparticle having a spherical structure with a diameter of 2-10 µm;
Step 3: the first recombinant collagen microparticle having a spherical structure were subjected to dry and thermal cross-linking for 2 h in a vacuum drying oven at 190°C to obtain a second recombinant collagen microparticle having a spherical structure with a high strength (implantable recombinant collagen microparticle); and
Step 4: 1.3 g of the second recombinant collagen microparticle having a spherical structure (implantable recombinant collagen microparticle), which was obtained by the two processes, i.e. self-assembly and dry and thermal cross-linking, was weighed, 9.7 g of water for injection was added, the microparticle were swollen for 12 h in a refrigerator at 4°C, and the mixture was stirred for 2 h at a rotating speed of 120 r/min to obtain a milky white opaque recombinant collagen gel with a concentration of 13% (wt) and a pH of 6.5.

### Example 6

Step 1: 50 g of a recombinant collagen raw material (a protein with an amino acid sequence as set forth in SEQ ID No: 1) was weighed, dissolved in 450 g of water for injection, and stirred for 4 hours at room temperature until it was completely dissolved and provided a colorless and transparent solution, and the pH was adjusted to be basic (pH = 8.87) to obtain a 10% (wt) recombinant collagen solution;
Step 2: the above solution was dried and granulated by a spray drier at 120°C to obtain a first recombinant collagen microparticle having a rod-like structure with a diameter of approximately 2-5 µm and various lengths;
Step 3: the first recombinant collagen microparticle having a rod-like structure were subjected to dry and thermal cross-linking for 2 h in a vacuum drying oven at 100°C to obtain a cross-linked second recombinant collagen microparticle having a rod-like structure (implantable recombinant collagen microparticle); and
Step 4: 1 g of the cross-linked second recombinant collagen microparticle having a rod-like structure (implantable recombinant collagen microparticle), which was obtained by the two processes, i.e. self-assembly and dry and thermal cross-linking, was weighed, 12.5 g of water for injection was added, the microparticles were swollen for 12 h in a refrigerator at 4°C, and the mixture was stirred for 2 h at a rotating speed of 120 r/min to obtain a colorless and transparent jelly-like recombinant collagen gel with a concentration of 8% (wt) and a pH of 7.

### Comparative Example 1

This example was basically the same as Example 1, except that the self-assembly step was not carried out.

### Comparative Example 2

This example was basically the same as Example 1, except that the dry and thermal cross-linking step was not carried out.

### Comparative Example 3

This example was basically the same as Example 1, except that the recombinant collagen was changed to a commercially available recombinant collagen (purchased from Xi'an DeNovo Hith Medical Technology Co., Ltd., batch No.: 2308B01).

### Effect Experiments

### 1. Appearance comparison test after implantable recombinant collagen microparticles were completely swollen into gels

(1) The implantable recombinant collagen microparticle was completely swollen with water to form a gel, and 2 ml of the gel was then taken and put into a transparent glass bottle, ensuring that the sample was uniform and had no bubbles. Under illumination by a standard sunlight lamp, the appearance of the material was photographed with a digital camera, and the transparency, color, and fluidity of the material were compared. After the implantable recombinant collagen microparticles prepared in Examples 1-6 and Comparative Examples 1-3 were completely swollen, the resulting gels were compared, and the appearance and state thereof were observed. The experimental results are as shown in Table 1.

**Table 1 Results of appearance comparison test after implantable recombinant collagen microparticles were completely swollen into gels**

| Appearance | Ex. 1 | Ex. 2 | Ex. 3 | Ex. 4 | Ex. 5 | Ex. 6 | Comp. Ex. 1 | Comp . Ex. 2 | Comp. Ex. 3 |
|---|---|---|---|---|---|---|---|---|---|
| Transparency | Opaque | Opaque | Opaque | Opaque | Opaque | Transparent | Opaque | Completely transparent | Opaque |
| Color | Milky white | Milky white | Milky white | Milky white | Milky white | Colorless | Milky white | Color less | Milky white |
| Fluidity | Low | Low | Low | Low | Low | Low | Relatively low | Relatively strong | Relatively low |

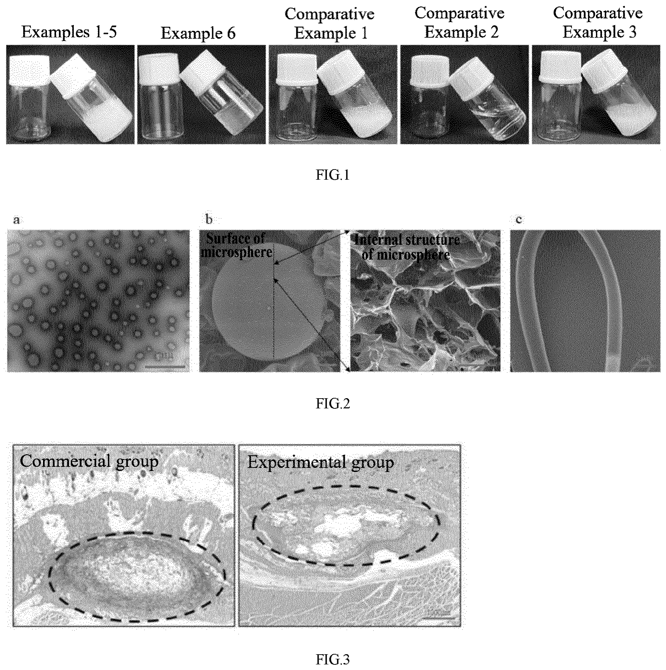

The above experimental results showed that the implantable recombinant collagen microparticles of Examples 1-5 were all in opaque milky white gel state and all had a low fluidity after being completely swollen with water, the implantable recombinant collagen microparticle of Example 6 became a colorless and transparent jelly-like gel after being completely swollen, Comparative Examples 1 and 3 were also in milky white gel state and both had a relatively low fluidity, and Comparative Example 2 was in a transparent and colorless sol state and had a strong fluidity. The experimental results are as shown in FIG. 1.

The recombinant collagen raw material was quickly dispersed into a nano-scale particle size in water, and the transmission electron microscopy image thereof is as shown in panel a of FIG. 2. The scanning electron microscope images of the surface and internal structure of the implantable recombinant collagen microparticle (spherical structure) obtained in the steps of Example 1 are as shown in panel b of FIG. 2. The scanning electron microscope image of the implantable recombinant collagen microparticle (rod-like structure) obtained in the steps of Example 6 is as shown in panel c of FIG. 2.

### 2. Biocompatibility test of the implantable recombinant collagen microparticle

(1) 24 healthy adult SD rats were randomly divided into 8 groups, with 3 rats in one group. The back was depilated using a depilatory agent so that the skin was prepared. The skin was cleaned with water, then the prepared skin of the rat was disinfected with tincture iodine, followed by deiodination with 75% ethanol. The implantable recombinant collagen microparticles prepared in Examples 1-5 and 1-3 were completely swollen with water to form gels and then injected into the rats in the 8 groups. Each rat was injected subcutaneously with 0.2 mL. The rats that had received injection were raised in the same environment for one week and then sacrificed by cervical dislocation. The epidermises that had received injection on the back of rats was taken for pathological analysis, and the inflammatory responses were observed. The experimental results are as shown in Table 2.

**Table 2 Inflammatory response test results**

| | Concentration of recombinant collagen solution (wt%) | Temperature in spray drier (°C) | Temperature in vacuum drying oven (°C) | Time in vacuum drying oven (h) | Concentration of recombinant collagen implant material (wt%) | Degree of inflammatory response (grades 1-5, with 1 being the mildest and 5 being the most severe) |
|---|---|---|---|---|---|---|
| Example 1 | 10 | 120 | 100 | 2 | 8 | 1 |
| Example 2 | 12 | 140 | 120 | 3 | 10 | 1 |
| Example 3 | 13 | 165 | 135 | 4 | 11 | 1 |
| Example 4 | 14 | 175 | 170 | 3 | 12 | 1 |
| Example 5 | 15 | 180 | 190 | 2 | 13 | 1 |
| Comparative Example 1 | 10 | / | 100 | 2 | 8 | 2 |
| Comparative Example 2 | 10 | 120 | / | / | 8 | 2 |
| Comparative Example 3 | 10 | 120 | 100 | 2 | 8 | 4 |

The above experimental results showed that the rats injected with the gels obtained after the implantable recombinant collagen microparticles of Examples 1-5were completely swollen, had basically no inflammatory responses produced, and the inflammatory responses in those injected with the gels obtained after the implantable recombinant collagen microparticles of Comparative Examples 1 and 2 were completely swollen, were also relatively mild; in contrast, the inflammatory responses in those injected with the gel obtained after the implantable recombinant collagen microparticle of Comparative Example 3 was completely swollen, were relatively severe.

After sampling, the skin injected with the experimental material was fixed in neutral formaldehyde. The hematoxylin-eosin staining method was then used for staining and photos were captured with an ordinary optical microscope, and pathological tissue analysis was performed. The experimental results are as shown in FIG. 3.

The experimental results showed that none of the rats in the example groups had obvious inflammatory responses one week after the rats were subcutaneously correspondingly injected; in contrast, the rats in the commercial group exhibited more severe inflammatory responses. This indicates that the implantable recombinant collagen microparticle prepared in the present application is a safe and non-toxic biomaterial with a good biocompatibility, so that the problems of poor biocompatibility and viral hazards of traditional biomaterials can be effectively solved.

### 3. Swelling performance test of the implantable recombinant collagen microparticle

Experimental method: The gel obtained after the implantable recombinant collagen microparticle obtained in the steps of Example 1 was completely swollen with water, was freeze-dried, and 5 mg of the freeze-dried gel was taken, immersed in purified water, and then swollen in an incubator at 37°C. Gels were taken out at 15 min, 30 min, 60 min, 120 min, and 180 min, respectively, and excess water on the surface was absorbed. The weight (w₁) of the gel was measured and compared with its initial weight (w₀). Swelling ratio = (w₁/w₀) × 100%.

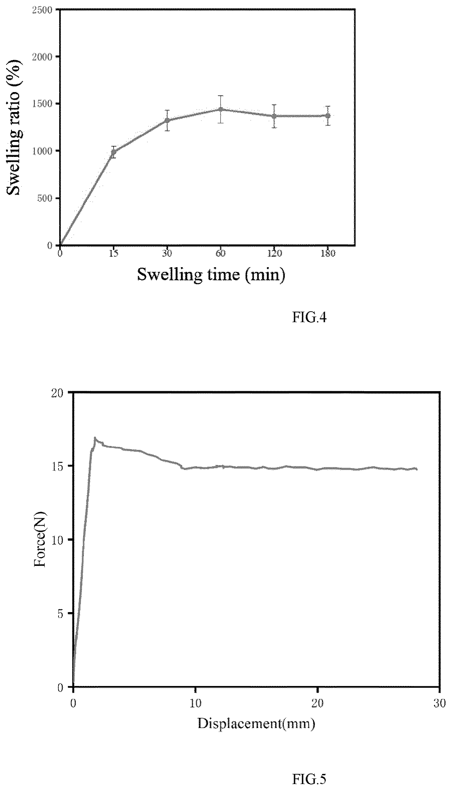

Experimental results: The swelling ratio of the freeze-dried gel reached 1400% after being immersed in water for 60 min, and then slowly decreased and stabilized at around 1300%. The experimental results are as shown in FIG. 4.

The experimental results showed that the implantable recombinant collagen microparticle had a relatively high swelling ratio. Since the material can take in growth factors by absorbing water and swelling, a higher swelling ratio can allow more aqueous solution to enter the material, which will help the material to absorb and preserve the growth factors.

### 4. Pushing force test of the gel obtained after the implantable recombinant collagen microparticle was completely swollen

Experimental method: The gel obtained after the implantable recombinant collagen microparticle obtained in Example 1 was completely swollen, was loaded into a 1 mL threaded syringe. It was ensured that the material was uniformly filled into the syringe, with bubbles or voids avoided. A 26G needle was installed, and it was ensured that the needle was tightly connected to the syringe. The syringe was fixed on a fixture of an electronic universal testing machine, and it was ensured that the syringe was vertical and stable. The loading rate on the electronic universal testing machine was set to 10 mm/min, and the material was completely pushed out of the syringe. A relationship plot of force versus displacement was obtained.

The experimental results showed that the pushing force for the gel was about 15N, indicating that the material has a good injection performance, and the low pushing force also reflected that the viscosity of the material was moderate, which will be neither too thin to hardly control nor too viscous to increase the difficulty of operation of a physician. The experimental results are as shown in FIG. 5.

### 5. Degradation test of the gels obtained after the implantable recombinant collagen microparticles were completely swollen

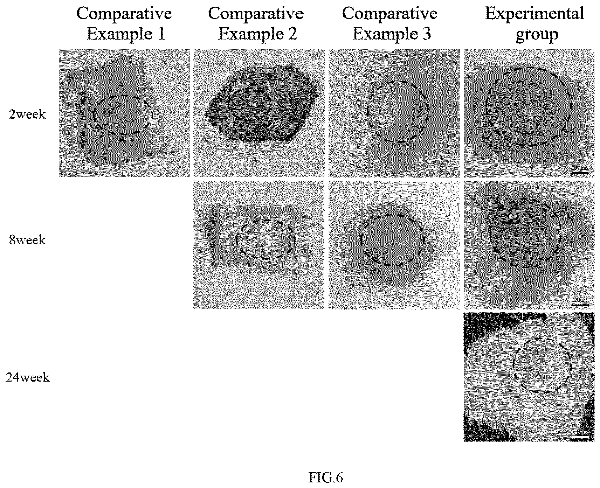

In the above experiment of Experimental 1, the rats in the group of Example 1 and the groups of Comparative Examples 1-3 were respectively sacrificed at 2, 8, and 24 weeks after feeding, and the epidermis with the injection site on the back was taken to observe the size of the material. The experimental results are as shown in FIG. 6.

The experimental results showed that after the gel in the group of Example 1 was subcutaneously injected into the rats, the material degraded to some extent subcutaneously over time, and a small amount of the material remained at 24 weeks. The gel in the group of Comparative Example 1 was injected subcutaneously into rats, and no material residue was observed upon sampling at week 2. The gels in the groups of Comparative Examples 2 and 3 were injected subcutaneously into rats, and no material residue was observed either upon sampling at week 8. It can be seen that the present application provides a gel obtained after an implantable recombinant collagen microparticle obtained by the method for preparing an implantable recombinant collagen microparticle is completely swollen. The gel has a relatively suitable degradation rate, can effectively solve the problem that traditional gel fillers can not degrade or degrade too fast, and can effectively solve the problem of poor biocompatibility of traditional biomaterials.

The above description of the embodiments is provided for those of ordinary skill in the art to understand and use the present disclosure conveniently. It is obvious that those familiar with the art can easily make various modifications to these examples and apply the general principles described herein to other examples without involving creative effort. Therefore, the present disclosure is not limited to the above examples, and improvements and modifications made by those skilled in the art according to the disclosure of the present disclosure without departing from the scope of the present disclosure shall all fall within the scope of protection of the present disclosure.

## Claims

1. A method for preparing an implantable recombinant collagen microparticle,
comprising:
(1) dissolving a recombinant collagen in water to obtain a recombinant collagen solution;
(2) drying and granulating the recombinant collagen solution to obtain a first recombinant collagen microparticle; and
(3) subjecting the first recombinant collagen microparticle to dry and thermal cross-linking to obtain a second recombinant collagen microparticle.

2. The method according to claim 1, wherein in step (2), the recombinant collagen solution is dried and granulated by using a spray drier;
preferably, the drying temperature in the spray drier is 100-200°C, more preferably 120-190°C.

3. The method according to claim 1, wherein step (2) further comprises adjusting the pH of the recombinant collagen solution to less than 7 or more than 7.

4. The method according to claim 1, wherein the first recombinant collagen microparticle has a rod-like structure or a spherical structure;
preferably, the diameter of the spherical structure is 2-10 µm, and the interior of the spherical structure is a porous network structure;
preferably, the diameter of the rod-like structure is 2-5 µm.

5. The method according to claim 1, wherein in step (3), the dry and thermal cross-linking is carried out in a vacuum drying oven;
preferably, the temperature for the dry and thermal cross-linking in the vacuum drying oven is 90-250°C,more preferably 100-240°C.

6. The method according to claim 5, wherein the dry and thermal cross-linking in the vacuum drying oven is carried out for 1-10 h, more preferably 2-9 h.

7. The method according to claim 1, wherein the concentration of the recombinant collagen solution is 5-20wt%, more preferably 8-15wt%.

8. The method according to claim 1, wherein the amino acid sequence of the recombinant collagen is represented by SEQ ID No: 1.

9. The method according to claim 1, wherein the method further comprises adding an aqueous phase to the second recombinant collagen microparticle to allow swelling, to obtain recombinant collagen microparticles in gel form.

10. The method according to claim 9, wherein after the aqueous phase is added to the second recombinant collagen microparticle, complete swelling is required;
preferably, the swelling time required for the complete swelling is 10-15 h.

11. The method according to claim 9, wherein the gel form is a milky white opaque gel or a colorless and transparent jelly-like gel.

12. An implantable recombinant collagen microparticle, wherein the amino acid sequence of the recombinant collagen in the recombinant collagen microparticle is represented by SEQ ID No: 1.

13. The implantable recombinant collagen microparticle according to claim 12, wherein the implantable recombinant collagen microparticle is prepared by the method according to any one of claims 1 to 11.

14. The implantable recombinant collagen microparticle according to claim 13, wherein the recombinant collagen microparticle exists in gel form upon addition of an aqueous phase.

15. An implantable recombinant collagen microparticle as defined in any one of claims 12 to 14 for use in the preparation of a medical material,
preferably, the medical material is used for medical aesthetic filling, soft tissue repair, or adhesion prevention.
